# EUROPEAN PATENT APPLICATION

(11) **EP 4 442 702 A1**
(43) Date of publication of application: **09.10.2024**
(21) Application number: 23819132.4
(22) Date of filing: 07.06.2023
(51) Int. Cl.: C07K 14/705, A61P 37/00

(54) **METHOD FOR TREATING MYASTHENIA GRAVIS WITH TACI-FC FUSION PROTEIN**

(30) Priority: 08.06.2022 CN 202210644750
(71) Applicant: RemeGen Co., Ltd., Shandong 264006 (CN)
(72) Inventor: FANG, Jianmin, Yantai, Shandong 264006 (CN); WANG, Wenxiang, Yantai, Shandong 264006 (CN)
(74) Representative: HGF
(86) International application number: PCT/CN2023/098731
(87) International publication number: WO 2023/236967

(57) **Abstract**

Provided are a drug, a dosage regimen, an administration interval and an administration mode for treating myasthenia gravis by means of using TACI-Fc fusion protein. Results show that the provided TACI-Fc fusion protein exhibits a good clinical efficacy and safety in the treatment of patients with myasthenia gravis.

## Description

### FIELD

The present disclosure relates to a drug for treating myasthenia gravis, a dosage regimen, an administration interval and an administration route.

### BACKGROUND

Myasthenia gravis (MG), as an autoimmune disease mediated by acetylcholine receptor antibody (AchR-Ab), dependent on cellular immunity, and involving complements, affects the postsynaptic membrane at the neuromuscular junction, resulting in impaired neuromuscular transmission and leading to weak contraction of skeletal muscles. MG in a very small proportion of patients is mediated by muscle-specific tyrosine kinase antibody (MuSK-Ab), and low-density lipoprotein receptor-related protein 4 antibody (LRP4-Ab). Skeletal muscles throughout the body of a MG patient can be affected. However, weakness of the extraocular muscles, pharyngeal muscles, or limb muscles may occur alone in the early course of the disease. Generally, MG starts with weakness in a muscle group, then gradually develops into weakness in additional muscle groups, and finally develops into a generalized muscle weakness. Some patients may suffer from short-term generalized muscle weakness, or even a myasthenic crisis, in which respiratory muscle weakness leads to respiratory distress, which may result in death of a patient in a short period of time. 80-90% of patients with MG have thymic abnormalities, mainly manifesting as thymus hyperplasia, cysts, necrosis, hypertrophy, or accompanied by various types of thymoma.

The incidence rate of myasthenia gravis in population is related to the age, gender and ethnicity, and varies in different countries and regions. MG can develop at all ages, with a higher incidence rate in women than in men before the age of 40; an equal incidence rate in men and women between the ages of 40 and 50; and a slightly higher incidence rate in men than in women after the age of 50. Childhood-onset MG is rare in western populations, but is prevalent in Asian countries, with about 50% of patients younger than 15 years old. According to statistics, 20 out of every 100,000 people in the United States suffer from myasthenia gravis, and the incidence rate of MG in China is about 0.68/100,000 people.

Myasthenia gravis is a lifelong disease for which there is currently no cure. The current treatment of MG mainly includes the following therapies: 1) Cholinesterase inhibitors such as pyridostigmine bromide and neostigmine: Cholinesterase inhibitors inhibit the degradation of acetylcholine, thereby increasing the availability of acetylcholine in the synapse. They usually have good effect on MG subgroups, but the effect varies greatly among individuals. In addition, cholinesterase inhibitors only treat symptoms rather than address the root cause, and they cannot be used as a single medication for a long term, and have poor effect on patients with MuSK-MG. 2) Immunosuppressants such as cyclophosphamide, cyclosporine A, mycophenolate mofetil, tacrolimus, methotrexate, and azathioprine: Immunosuppressants improve muscle strength by inhibiting the production of abnormal antibodies, but may cause serious side effects. 3) Intravenous immunoglobulin: Intravenous immunoglobulin is mainly used in MG patients with acute disease progression and in preoperative preparation. It can be used in combination with slow-acting immunosuppressive drugs or high-dose glucocorticosteroids that may induce myasthenic crisis, and usually shows an onset of action about 5-10 days after administration, with an effect lasting about 2 months. 4) Plasma exchange: Plasma exchange is a method that removes harmful antibodies from plasma and replace with high-quality plasma or alternative plasma using an instrument. It is mainly used in the acute progression phase of the disease, in patients with myasthenic crisis, before thymectomy, in perioperative treatment, and in the early stage of immunosuppressive therapy. It can temporarily relieve the symptoms of patients with myasthenia gravis, but its effect does not last more than 2 months without additional auxiliary treatment. Repeated treatment by this method for a long term does not increase the long-term effect. 5) Thymectomy: Thymectomy is suitable for patients who develop generalized myasthenia gravis between the ages of 16 and 60 years and have no contraindications for surgery. 6) Other therapies, for example, respiratory muscle training and strength exercises in patients with mild MG. Overall, common treatments for MG have certain risks and limitations, and unsatisfactory therapeutic effects. MG is an antibody-mediated disease in terms of pathogenesis, so it is currently an urgent need for the treatment of MG to develop new effective targeted drugs.

FDA has approved three targeted biologic formulations for the treatment of MG in recent years, including eculizumab (on 23 October, 2017), efgartigimod (on 17 December, 2021) and ravulizumab (on 28 April, 2022).

Eculizumab (trade name: Soliris) is a recombinant human monoclonal antibody that has an inhibitory effect on complement component C5 of terminal phase and can prevent membrane attack complex (MAC) production mediated by anti-AChR antibody. In a study on refractory MG with 26 weeks of follow-up, eculizumab was administered at an amount of 900 mg on day 1 and weeks 1, 2, and 3; 1200 mg on week 4; and 1200 mg every two weeks thereafter as maintenance administration. Vaccination against *Neisseria meningitidis* was necessary. The results of the study show that, from baseline to the end of follow-up, the treatment group was only statistically different from the placebo group in the secondary endpoint (defined as the need for rescue therapy for MG exacerbation), while there was no difference in the primary endpoint (defined as MG-activities of daily living score, MG-ADL). The treatment had an onset of action within 4-8 weeks. The most common adverse events in the trial were headache and upper respiratory tract infection. Although eculizumab is approved for the treatment of patients with refractory MG, the extremely high price (at least 450,000 euros per year) may be a major limitation to its use.

Ultomiris (Ravulizumab) is a long-acting formulation improved from the first generation of C5 monoclonal antibody eculizumab, which has an extended half-life compared to eculizumab, and is now approved for the treatment of adult patients with anti-acetylcholine receptor (AChR)-antibody-positive generalized myasthenia gravis (gMG). In a global phase 3 randomized, double-blind, placebo-controlled, multi-center, 26-week study, evaluated was the safety and efficacy of ravulizumab in adults with gMG who were not previously treated with a complement inhibitor medicine. The study enrolled 175 patients across North America, Europe, Asia-Pacific and Japan. To enter the study, the patients were required to have a confirmed MG diagnosis at least 6 months prior to the screening visit, a positive serologic test for anti-AChR antibodies, an MG-ADL total score of at least 6 at study entry, and a clinical classification of II to IV by Myasthenia Gravis Foundation of America (MGFA) at screening. In the study, the patients were administered with ravulizumab or placebo randomly at a ratio of 1:1 for a total of 26 weeks. The patients were administered with a single loading amount based on body weight on day 1, and then a regular maintenance amount based on body weight every 8 weeks from day 15. The change in the MG-ADL total score (primary endpoint) on week 26 compared with baseline was evaluated along with multiple secondary endpoints of evaluating improvement in disease-related and quality of life measures. As shown in the resulting data, the study met its primary endpoint, with a statistically significant change in MG-ADL total score (ravulizumab: -3.1, placebo: -1.4, treatment difference: -1.6, p<0.001) from baseline to week 26 in ravulizumab group compared to placebo group. In addition, for the prospectively-defined secondary endpoints: from baseline to week 26, in aspect of quantitative myasthenia gravis (QMG) total score (a physician-administered assessment of MG clinical severity) and the proportion of patients who achieved an improvement of at least 5 points in QMG, ravulizumab also demonstrated clinically meaningful and statistically significant improvements (p<0.001 and p=0.005, respectively). Nearly three times as many patients receiving ravulizumab experienced an improvement of at least 5 points in their QMG score compared to patients receiving placebo (30.0% vs 11.3%). These improvements in MG-ADL and QMG scores were observed as early as week 1 and were sustained through week 26. During the randomized controlled period, adverse events were comparable between the ravulizumab and placebo groups. The most frequently observed adverse events were headache (ravulizumab: 18.6%; placebo: 25.8%), diarrhea (ravulizumab: 15.1%; placebo: 12.4%) and nausea (ravulizumab: 10.5%; placebo: 10.1%). The most frequently observed serious adverse events were MG crisis (ravulizumab: 1.2%) and MG worsening (placebo: 3.4%). In addition, ravulizumab also has the problem of being expensive.

Vyvgart (efgartigimod) is an antibody fragment that binds to the neonatal Fc receptor (FcRn), which can prevent FcRn from recycling immunoglobulin G (IgG) back into the blood. This medication causes a reduction in the overall level of IgG, including the abnormal AChR antibodies that are present in myasthenia gravis. Currently, this medication is approved for the treatment of generalized myasthenia gravis (gMG) in adults who are tested positive for the anti-acetylcholine receptor (AChR) antibody. The safety and efficacy of Vyvgart were evaluated in a 26-week clinical study on 167 patients with myasthenia gravis. The patients were administered with either Vyvgart or placebo randomly. The study shows that in the evaluation of the impact of myasthenia gravis on daily functions, Vyvgart had an effect on the patients with myasthenia gravis with more antibodies during the first cycle of the treatment (68%) compared to those who received placebo (30%). In terms of muscle weakness, more patients administered with Vyvgart showed a therapeutic effect compared to those who administered with placebo. The most common side effects associated with the use of Vyvgart include respiratory tract infections, headache, and urinary tract infections. As Vyvgart causes a reduction in IgG levels, the risk of infections may increase.

Since the 1960s, myasthenia gravis has been considered a "second cancer", with patients suffering from lifelong fatigue and weakness, which gradually progress to death. Despite the rapid development of MG treatment in recent years, there is a huge unmet clinical need for myasthenia gravis treatment in China and worldwide.

### SUMMARY

Surprisingly, the TACI-Fc fusion protein provided by the present invention exhibits significant therapeutic effect in the treatment of a patient with myasthenia gravis.

Specifically, the present invention provides a method for treating myasthenia gravis, comprising administering a therapeutically effective amount of a TACI-Fc fusion protein to a subject in need thereof, wherein the TACI-Fc fusion protein comprises:
(i) a TACI extracellular region or a fragment thereof binding to Blys and/or APRIL; and
(ii) a fragment of a constant region of a human immunoglobulin.

Specifically, the present invention provides a method for treating myasthenia gravis, comprising (1) determining whether a subject has received a standard treatment regimen for myasthenia gravis, and (2) administering to the subject an effective amount of a TACI-Fc fusion protein if the subject has received a standard treatment regimen for myasthenia gravis. The standard treatment regimen for myasthenia gravis includes: a cholinesterase inhibitor alone, a cholinesterase inhibitor in combination with a glucocorticoid or an additional immunosuppressant specified by the regimen, a glucocorticoid in combination with an additional immunosuppressant specified by the regimen, and a cholinesterase inhibitor in combination with a glucocorticoid and an additional immunosuppressant specified by the regimen.

Specifically, the present invention provides use of a TACI-Fc fusion protein in the manufacture of a medicament for the treatment a subject with myasthenia gravis.

Preferably, the TACI extracellular region or the fragment thereof binding to Blys and/or APRIL comprises an amino acid sequence set forth in SEQ ID NO: 1.

SEQ ID NO: 1

SRVDQEERFP QGLWTGVAMR SCPEEQYWDP LLGTCMSCKT ICNHQSQRTC AAFCRSLSCR 60
KEQGKFYDHL LRDCISCASI CGQHPKQCAY FCENKLRSPV NLPPEL 106

Further preferably, the constant region of the human immunoglobulin comprises an amino acid sequence set forth in SEQ ID NO: 2 or an amino acid sequence having at least 90%, at least 91%, at least 92%, at least 93%, at least 95%, at least 96%, at least 97%, at least 98%, or at least 99% identity to SEQ ID NO: 2.

SEQ ID NO: 2

DKPHTCPLCP APELLGGPSV FLFPPKPKDT LMISRTPEVT CVVVDVSHED PEVKFNWYVD 60
GVEVHNAKTK PREEQYNSTY RVVSVLTVLH QDWLNGKEYK CKVSNKALPA PIEKTISKAK 120
GQPREPQVYT LPPSRDELTK NQVSLTCLVK GFYPSDIAVE WESNGQPENN YKATPPVLDS 180
DGSFFLYSKL TVDKSRWQQG NVFSCSVMHE ALHNHYTQKS LSLSPGK 227

Further preferably, the constant region of the human immunoglobulin comprises one or more amino acids with a modification, and wherein the amino acid is selected from 3, 8, 14, 15, 17, 110, 111 or 173 position of SEQ ID NO: 2, wherein the modification is preferably substitution, deletion or insertion of amino acid.

Further preferably, the substitution is selected from the group consisting of P3T, L8P, L14A, L15E, G17A, A110S, P111S and A173T.

Further preferably, the constant region of the human immunoglobulin comprises an amino acid sequence set forth in SEQ ID NO: 3.

SEQ ID NO: 3

DKTHTCPPCP APEAEGAPSV FLFPPKPKDT LMISRTPEVT CVVVDVSHED PEVKFNWYVD 60
GVEVHNAKTK PREEQYNSTY RVVSVLTVLH QDWLNGKEYK CKVSNKALPS SIEKTISKAK 120
GQPREPQVYT LPPSRDELTK NQVSLTCLVK GFYPSDIAVE WESNGQPENN YKTTPPVLDS 180
DGSFFLYSKL TVDKSRWQQG NVFSCSVMHE ALHNHYTQKS LSLSPGK 227

Further preferably, the human immunoglobulin is IgG1.

Further preferably, the TACI-Fc fusion protein is Telitacicept and has an amino acid sequence set forth in SEQ ID NO: 4.

SEQ ID NO: 4

SRVDQEERFP QGLWTGVAMR SCPEEQYWDP LLGTCMSCKT ICNHQSQRTC AAFCRSLSCR 60
KEQGKFYDHL LRDCISCASI CGQHPKQCAY FCENKLRSPV NLPPELDKTH TCPPCPAPEA 120

EGAPSVFLFP PKPKDTLMIS RTPEVTCVVV DVSHEDPEVK FNWYVDGVEV HNAKTKPREE 180
QYNSTYRVVS VLTVLHQDWL NGKEYKCKVS NKALPSSIEK TISKAKGQPR EPQVYTLPPS 240
RDELTKNQVS LTCLVKGFYP SDIAVEWESN GQPENNYKTT PPVLDSDGSF FLYSKLTVDK 300
SRWQQGNVFS CSVMHEALHN HYTQKSLSLS PGK 333

Further preferably, the TACI-Fc fusion protein is administered at a dose of 0.1 to 10 mg/kg each time.

Further preferably, the TACI-Fc fusion protein is administered at a dose of 160 to 240 mg each time, more preferably 160 mg or 240 mg each time.

Further preferably, the TACI-Fc fusion protein is administered 2-4 times per month, or is administered 2 times, 3 times, or 4 times per month.

Further preferably, the TACI-Fc fusion protein is administered once a week.

Further preferably, the treatment lasts for about 2-50 weeks. More preferably, the treatment lasts for 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 41 weeks, 42 weeks, 43 weeks, 44 weeks, 45 weeks, 46 weeks, 47 weeks, 48 weeks, 49 weeks, or 50 weeks.

Further preferably, the TACI-Fc fusion protein is administered subcutaneously, intramuscularly or intravenously, into a site on a thigh, an abdomen or an upper arm. In some specific embodiments, the TACI-Fc fusion protein is injected subcutaneously, intramuscularly or intravenously.

Further preferably, the TACI-Fc fusion protein is injected at the same site or at different sites per injection. In some specific embodiments, the TACI-Fc fusion protein is injected at the same site per injection. In some other specific embodiments, the TACI-Fc fusion protein is injected at different sites per injection.

Further preferably, the myasthenia gravis is generalized myasthenia gravis (gMG).

Further preferably, the myasthenia gravis is acetylcholine receptor antibody (AchR-Ab)-positive.

Further preferably, the myasthenia gravis is muscle-specific receptor tyrosine kinase antibody (MuSK-Ab)-positive.

Further preferably, the myasthenia gravis is classified as class II or III according to the Myasthenia Gravis Foundation of America clinical classification.

Further preferably, the myasthenia gravis has a quantitative myasthenia gravis score (QMG) of ≥ 8, and each of at least 4 items has a score of at least 2.

Further preferably, the subject is an adult diagnosed with generalized myasthenia gravis classified as class II or III according to the Myasthenia Gravis Foundation of America (MGFA) clinical classification, who is AchR-Ab-positive or MuSK-Ab-positive, previously received standard treatment, and has a QMG of ≥ 8, and each of at least 4 items has a score of at least 2 at screening.

Generalized myasthenia gravis is an autoimmune disease that affects the neuromuscular junction and causes weakness. The disease severity of patients can be quantified using scale score, such that the condition of patients can be more intuitively evaluated, which is helpful for clinical observation and evaluation of drug efficacy. In the present invention, the efficacy of treatment is evaluated using QMG and a clinical absolute scoring scale for MG.

QMG mainly measures muscular strength and endurance of patients, which was introduced in 2000 by the Myasthenia Gravis Foundation in the United States, and requires the use of assistive equipment, including a handheld dynamometer for grip strength and a stopwatch timer. QMG consists of 13 items: diplopia, ptosis, facial muscle strength, swallowing, speech, right arm outstretched, left arm outstretched, vital capacity (VC, % predicted), head lifted, right hand grip, left hand grip, right leg outstretched, and left leg outstretched, with a total score from 0 to 39. The higher the score, the more severe the disease. QMG is evaluated 8 hours after the last administration of a cholinesterase inhibitor.

The clinical absolute scoring scale for myasthenia gravis mainly evaluates the severity of muscle weakness and fatigability of the muscle groups affected by myasthenia gravis. This scale consists of the following 8 items: upper eyelid muscle weakness, upper eyelid fatigue test, level of eyeball horizontal movement, upper limb fatigue test, lower limb fatigue test, facial muscle strength, chewing and swallowing function, and respiratory muscle function, with a total score from 0 to 60. The higher the score, the more severe the disease. The clinical absolute scoring scale for myasthenia gravis is evaluated 8 hours after the last administration of a cholinesterase inhibitor.

The Myasthenia Gravis Foundation of America (MGFA) strongly recommended the use of the QMG scale for prospective clinical studies related to MG in 2000. The clinical absolute scoring scale for myasthenia gravis reflects the severity of muscle weakness and fatigability of the affected muscle groups in patients with MG by using absolute clinical scores, which is sensitive to changes in the clinical condition, and is an ideal method for quantifying changes in the condition of patients with MG.

Further preferably, the subject is an adult or a child.

Further preferably, the subject has previously and stably received a standard treatment regimen for myasthenia gravis. In some specific embodiments, the standard treatment regimen includes: a cholinesterase inhibitor alone, a cholinesterase inhibitor in combination with a glucocorticoid or an additional immunosuppressant specified by the regimen, a glucocorticoid in combination with an additional immunosuppressant specified by the regimen, and a cholinesterase inhibitor in combination with a glucocorticoid and an additional immunosuppressant specified by the regimen.

The TACI-Fc fusion protein provided by the present invention exhibits an unexpected clinical efficacy and a good safety in treating myasthenia gravis.

### BRIEF DESCRIPTION OF DRAWINGS

FIG. 1 shows the diachronic changes in QMG score compared with baseline.
FIG. 2 shows the proportion of subjects with different degrees of improvement in the QMG score at each follow-up visit.
FIG. 3 shows diachronic changes in clinical absolute score for MG compared with baseline.
FIG. 4 shows diachronic change rate of immunoglobulin G (IgG) level compared with baseline.
FIG. 5 shows diachronic change rate of immunoglobulin A (IgA) level compared with baseline.
FIG. 6 shows diachronic change rate of immunoglobulin M (IgM) level compared with baseline.
FIG. 7 shows diachronic change rate of B lymphocyte (CD19⁺) count compared with baseline.

### DETAILED DESCRIPTION

Unless otherwise defined, all scientific and technical terms used herein have the same meaning as understood by those of ordinary skill in the art. For definitions and terms in the art, those skilled can make a reference to Current Protocols in Molecular Biology (Ausubel).

The three-letter codes and one-letter codes of amino acids used in the present invention are as described in J. biol. chem, 243, p3558 (1968).

The present invention provides use of a TACI (transmembrane activator and calcium modulator and cyclophilin ligand interactor)-immunoglobulin fusion protein (TACI-Fc fusion protein) in the treatment of myasthenia gravis. The TACI-immunoglobulin fusion protein provided by the present invention comprises: 1) a polypeptide containing a domain that is at least partially identical to the extracellular domain of TACI or a fragment thereof capable of binding BlyS and/or APRIL; and 2) a fragment of a constant region of a human immunoglobulin. U.S. Patent Nos. 5,969,102, 6,316,222, and 6,500,428 and U.S. Patent Applications 09/569,245 and 09/627,206 (the contents thereof are incorporated herein by reference), disclose the extracellular domain of TACI and specific fragments of TACI extracellular domain capable of interacting with TACI ligands, wherein the TACI ligands include BlyS and APRIL. Chinese Patent Publication No. CN101323643A discloses a TACI-Fc fusion protein comprising a fragment of the 13th-118th amino acids of the TACI extracellular domain.

The immunoglobulin moiety of the TACI-immunoglobulin fusion protein provided by the present invention is preferably IgG1, which may comprises a heavy chain constant region, e.g., a human heavy chain constant region. The preferred IgG1 heavy chain constant region of the present invention comprises an IgG1 Fc fragment containing CH2 and CH3 domains, which may be a wild-type IgG1 Fc fragment or a mutated IgG1 Fc fragment.

The TACI-Fc fusion protein may be administered to a subject in need thereof by any appropriate routes, including but not limited to, intravenously, intramuscularly, or subcutaneously.

The TACI-Fc fusion protein provided by the present invention is prepared into a preparation and stored in lyophilized form, aseptic form and solution. Such preparation may comprise an additional active ingredient, an excipient or additional substances such as sodium chloride, phosphate buffer and sodium hydroxide. The TACI-Ig preparation can be administered to a subject in need thereof in combination with an additional drug, and can be administered before, simultaneously with, or after an additional treatment.

The term "treatment" used in the present invention is related to a given disease or condition, including but not limited to: inhibiting the disease or symptom, such as preventing the development of the disease or symptoms; alleviating the disease or symptoms, such as causing the regress of the disease or symptoms; or alleviating the symptoms caused by the disease or symptoms, such as alleviating, preventing or treating the symptoms caused by the disease or symptoms.

Embodiments of the present invention will be described in detail below in conjunction with examples. However, those skilled in the art will understand that the following examples are only intended to illustrate the present invention, and should not be construed as limiting the scope of the present invention.

### Example 1 Clinical trial of Telitacicept in treating myasthenia gravis

### 1. Methods

This multi-center, randomized, open clinical study aimed to preliminarily evaluate the efficacy and safety of telitacicept in the treatment of patients with generalized myasthenia gravis. Included were subjects with generalized MG of class II/III in MGFA clinical classification, who were AChR-Ab-positive or MuSK-Ab-positive, received standard treatment but still had a QMG score of ≥8. These subjects were randomly assigned to telitacicept 160 and 240 mg groups, and the tested drug was administered once weekly for a total of 24 administrations.

### 2. Inclusion criteria

The subjects were adults diagnosed with generalized myasthenia gravis classified as class II or III according to the Myasthenia Gravis Foundation of America (MGFA) clinical classification, who were AchR-Ab-positive or MuSK-Ab-positive, received standard treatment, and had a QMG of ≥ 8, and each of at least 4 items has a score of at least 2 at screening.

### 3. Treatment

These subjects were randomly assigned to either telitacicept 160 group or telitacicept 240 mg group. Telitacicept was injected subcutaneously into a thigh, abdomen, or upper arm once a week for a total of 24 injections. Each injection was given at a different injection site.

### 4. Evaluation criteria

### 4.1 Efficacy indicators

### 4.1.1 Primary endpoint indicator

Mean change in QMG score on week 24 compared with baseline.

### 4.1.2 Secondary endpoint indicator

Mean change in clinical absolute score for myasthenia gravis on weeks 12 and 24 compared with baseline. Mean change in QMG score on week 12 compared with baseline.

### 4.1.3 Safety endpoints

### Frequency and severity of adverse events

### 5. Demographics and baseline characteristics

There were 3 analysis sets in this study.
- Full analysis set (FAS) was a set of all subjects that were randomly grouped and administered with the tested drug at least once.
- Per protocol set (PPS) was a set of subjects who were compliant with the protocol, wherein the compliance includes the completion of the treatment regimen, the availability of measurements of the primary endpoint, and the absence of any major protocol violations.
- Safety set (SS) is a set of actual data of subjects who received at least one treatment and had record of safety indicators. The incidence rate of adverse events was calculated with the number of cases in the safety set as the denominator.

41 patients were screened for this trial and 29 were randomly enrolled. All 29 cases were observed in the 24-week trial and enrolled in the full analysis set (FAS), per protocol set (PPS) and safety set (SS), with 14 FAS cases, 14 PPS cases, and 14 SS cases in the telitacicept 160 mg group, and 15 FAS cases, 15 PPS cases, and 15 SS cases in the telitacicept 240 mg group.

The average age of the 29 subjects was 44.1 years old, and 55.2% of them were female. All subjects were positive for AChR antibodies, wherein 28 subjects were tested for MuSK-Ab levels (1 subject in the telitacicept 160 mg group was not tested), and all showed negative. The subjects had a median disease duration of 25.4 months, an average QMG score of 18.9, and an average MG clinical absolute score of 24.0. According to MGFA clinical classification, 16 subjects (55.2%) were classified as class II, and 13 subjects (44.8%) were classified as class III. Nine subjects (31.0%) were treated with a cholinesterase inhibitor alone, 13 subjects (44.8%) were treated with a cholinesterase inhibitor in combination with a glucocorticoid/an additional immunosuppressant specified by the regimen, and 7 subjects (24.1%) were treated with a cholinesterase inhibitor in combination with a glucocorticoid and an additional immunosuppressant specified by the regimen.

### 6. Efficacy

### 6.1 Efficacy analysis

Efficacy analysis was conducted on FAS and PPS. Since all 29 subjects randomly grouped in this study were included in the FAS and PPS, and the FAS and PPS analyses were the same, the following description of the efficacy outcomes no longer distinguishes between the FAS and PPS.

### 6.2 Primary endpoint

The primary endpoint of this study was the mean change in QMG score on week 24 compared with baseline. The analysis was performed using analysis of covariance (ANCOVA), with groups as fixed effect and the baseline value as a covariate.

On week 24, compared with baseline, the change in QMG scores (mean± SD) of the subjects were -7.7±5.34 and -9.6±4.29 in the telitacicept 160 and 240 mg groups, respectively (Table 1).

**Table 1 Change in QMG score on week 24 compared with baseline**

| | Telitacicept 160 mg group (N=14) | Telitacicept 240 mg group (N=15) |
|---|---|---|
| Mean± SD | -7.7±5.34 | -9.6±4.29 |
| Median | -5.5 | -10.0 |
| 95%CI | -10.8 to -4.6 | -12.0 to -7.2 |
| Min-Max | -16 to -1 | -20 to -3 |

The difference in least squares means (LSMEANS) of the change in QMG scores on week 24 compared with baseline between the telitacicept 160 mg group and the telitacicept 240 mg group was compared using ANCOVA. The LSMEANS of the change in QMG score on week 24 compared with baseline was -7.96 (95% CI: -10.07 to -5.85) in the telitacicept 160 mg group and -9.37 (95% CI: -11.40 to -7.33) in the telitacicept 240 mg group. The difference in LSMEANS between the two groups was -1.40 (95% CI: -4.34 to 1.53). See Table 2.

**Table 2 LSMEANS of the change in QMG score on week 24 compared with baseline**

| **Group** | **LSMEANS** | **95%CIL** | **95%CIU** |
|---|---|---|---|
| Telitacicept 160 mg group | -7.96 | -10.07 | -5.85 |
| Telitacicept 240 mg group | -9.37 | -11.40 | -7.33 |
| Telitacicept 240 mg group-160 mg group | -1.40 | -4.34 | 1.53 |

### 6.3 Secondary endpoint

### 6.3.1 Mean change in QMG score on week 12 compared with baseline

The mean change in QMG scores on week 12 compared with baseline was one of the secondary efficacy indicators in this study, and differences between groups were compared using t-tests.

On week 12, compared with baseline, the change in QMG scores (mean ±SD) of the subjects were -5.8±5.85 and -9.5±5.03 in the telitacicept 160 and 240 mg groups, respectively. See Table 3.

**Table 3 Change in QMG score on week 24 compared with baseline**

| | Telitacicept 160 mg group (N=14) | Telitacicept 240 mg group (N=15) |
|---|---|---|
| Mean± SD | -5.8±5.85 | -9.5±5.03 |
| Median | -5.0 | -10.0 |
| 95%CI | -9.2 to -2.4 | -12.3 to -6.7 |
| Min-Max | -15 to 5 | -21 to -2 |

### 6.3.2 Mean change in MG clinical absolute scores on weeks 12 and 24 compared with baseline

The mean change in MG clinical absolute scores on weeks 12 and 24 compared with baseline was one of the secondary efficacy indicators in this study, and differences between groups were compared using t-tests.

On week 12, compared with baseline, the change in MG clinical absolute scores (mean ±SD) of the subjects was -10.4±4.40 in the telitacicept 160 mg group, and -11.2±9.88 in the telitacicept 240 mg group. On week 24, compared with baseline, the change in MG clinical absolute scores (mean ± SD) of the subjects was -13.8±7.30 in the telitacicept 160 mg group and -14.1±9.78 in the telitacicept 240 mg group. See Table 4.

**Table 4 Change in MG clinical absolute scores on weeks 12 and 24 compared with baseline**

| | Telitacicept 160 mg group (N=14) | Telitacicept 240 mg group (N=15) |
|---|---|---|
| Change in MG clinical absolute scores on week 12 compared with baseline | | |
| Mean± SD | -10.4±4.40 | -11.2±9.88 |
| Median | -10.0 | -13.0 |
| 95%CI | -12.9 to -7.8 | -16.7 to -5.7 |
| Min-Max | -18 to -3 | -31 to 5 |

| Change in MG clinical absolute scores on week 24 compared with baseline | | |
|---|---|---|
| Mean± SD | -13.8±7.30 | -14.1±9.78 |
| Median | -11.5 | -13.0 |
| 95%CI | -18.0 to -9.6 | -19.5 to -8.7 |
| Min-Max | -28 to -2 | -33 to 1 |

### 6.4 Additional efficacy outcomes

### 6.4.1 Diachronic changes in QMG score compared with baseline

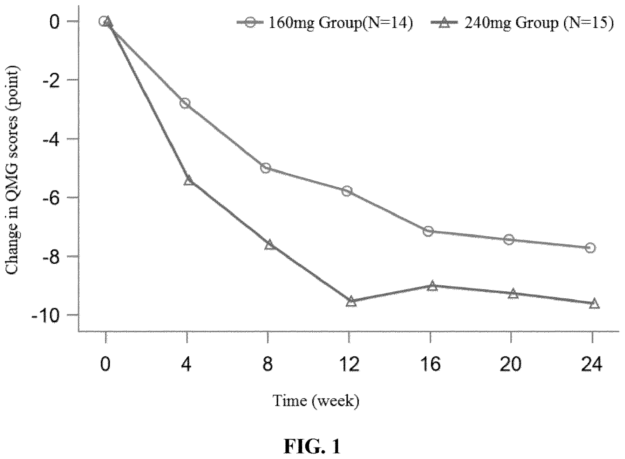

FIG.1 shows the diachronic changes in QMG score compared with baseline. From week 4, the subjects in both the telitacicept 160 mg group and the telitacicept 240 mg group had a significant decrease in QMG scores compared with baseline, which lasted till week 24.

### 6.4.2 Proportion of subjects with improvement in the QMG score

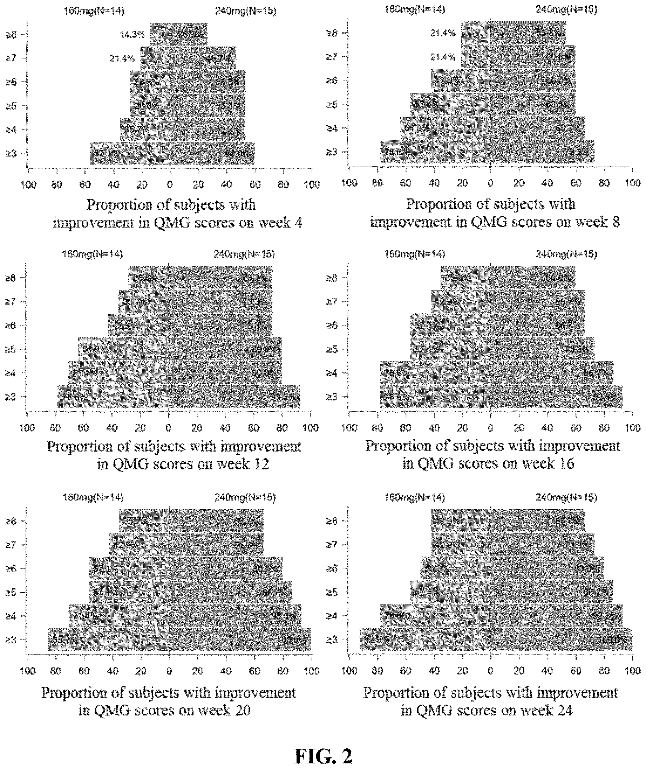

At each follow-up visit, the proportion of subjects exhibiting different degrees of improvement defined by the QMG score (i.e., a decrease in QMG scores by ≥3, ≥4, ≥5, ≥6, ≥7, and ≥8) is shown in FIG. 2.

From the follow-up visit on week 4 to the follow-up visit on week 24, the proportion of subjects with different degrees of improvement in QMG scores in the telitacicept 240 mg group was higher than those in the 160 mg group. On week 24, in the telitacicept 160 mg group and telitacicept 240 mg group, the proportions of subjects with improvements in QMG scores are as follows: ≥3 were 92.9% and 100.0%, respectively; ≥4 were 78.6% and 93.3%, respectively; ≥5 were 57.1%, and 86.7%, respectively; ≥6 were 50.0%, and 80.0%, respectively; ≥7 were 42.9%, and 73.3%, respectively; and ≥8 were 42.9% and 66.7%, respectively.

### 6.4.3 Diachronic changes in clinical absolute score for MG compared with baseline.

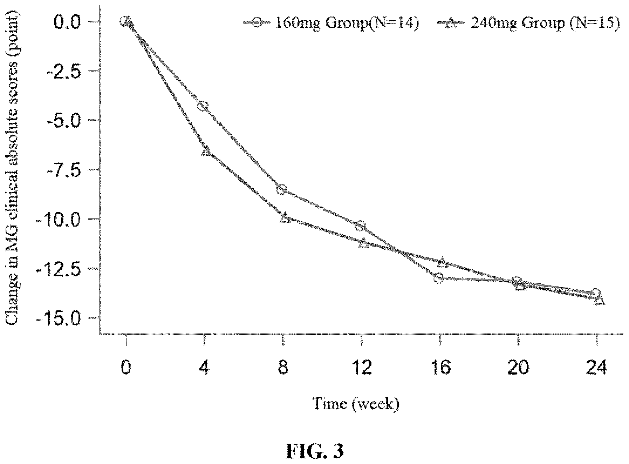

FIG. 3 shows diachronic changes in clinical absolute score for MG compared with baseline. From week 4, subjects in both the telitacicept 160 mg group and the telitacicept 240 mg group had a significant decrease in MG clinical absolute scores from baseline, which continued through week 24.

### 6.4.4 Immunological indicators

### 6.4.4.1 Immunoglobulins (IgG, IgA, and IgM)

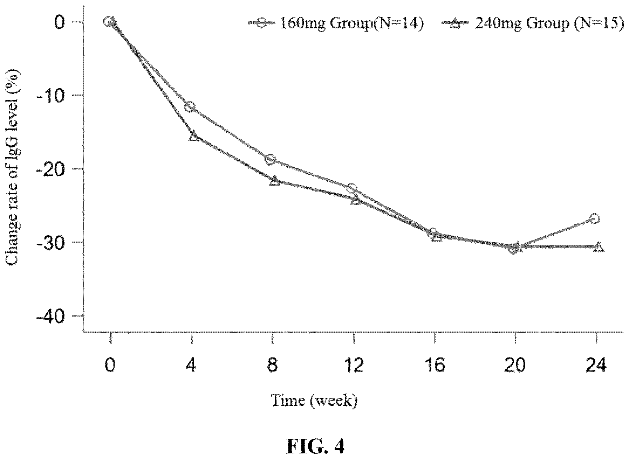

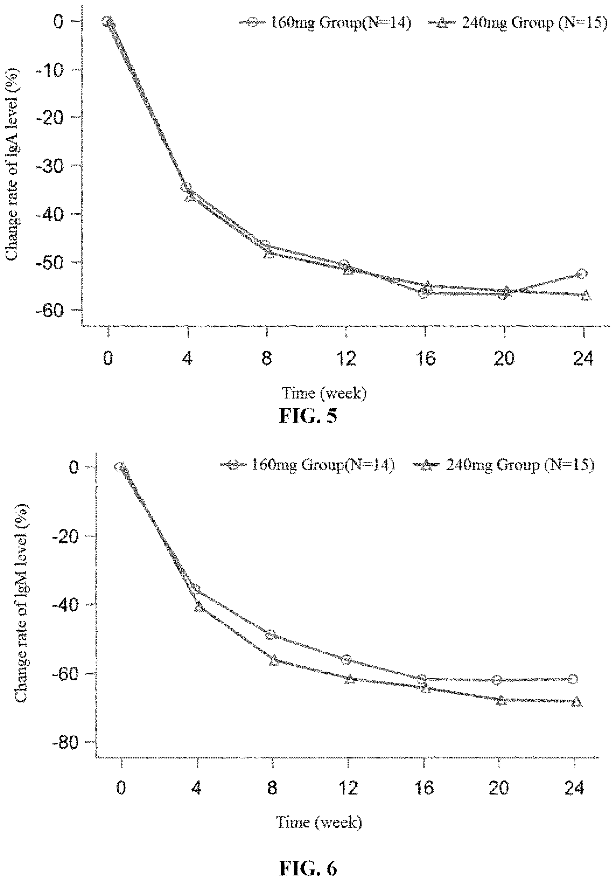

The analysis results of the diachronic change rate of immunoglobulin (IgG, IgA, and IgM) levels compared with baseline are shown in FIGs 4-6. A significant decrease in IgG, IgA, and IgM levels (g/L) showed in subjects in both the telitacicept 160 mg group and the telitacicept 240 mg group from week 4 to week 24.

On week 24, the change rate (%) (mean± SD) of IgG levels of subjects compared with baseline was -26.827±17.127 in the telitacicept 160 mg group and -30.595±13.037 in the telitacicept 240 mg group.

On week 24, the change rate (%) (mean± SD) of IgA levels of subjects compared with baseline was -52.475±17.954 in the telitacicept 160 mg group and -56.850±14.781 in the telitacicept 240 mg group.

On week 24, the change rate (%) (mean± SD) of IgM levels of subjects compared with baseline was -61.747±17.299 in the telitacicept 160 mg group and -68.203±10.821 in the telitacicept 240 mg group.

### 6.4.4.2 CD19⁺ B Cells

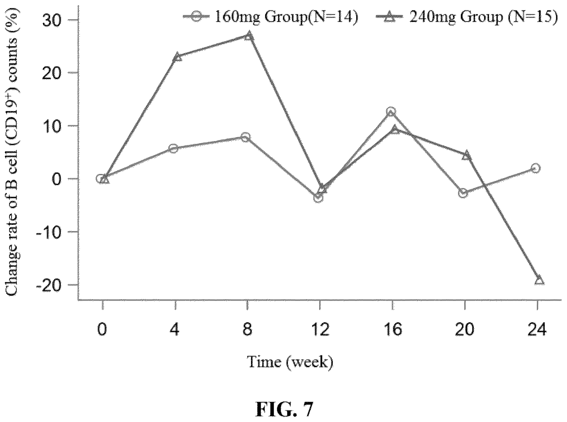

FIG. 7 shows diachronic change rate of B lymphocyte (CD19⁺) count compared with baseline. After administration of telitacicept 240 mg, compared with baseline, the median value of CD19⁺ B cell count of the subjects showed a trend of increasing and then decreasing. On week 24, the median value of CD19⁺ B cell count of the subjects significantly decreased compared with baseline. In contrast, although the median value of CD19⁺ B-cell count of the subjects administered with telitacicept 160 mg fluctuated, it had no significant trend of change.

### 6.5 Efficacy analysis

In this study, the treatment of patients with generalized myasthenia gravis with telitacicept 160 mg and 240 mg demonstrated consistently improved clinical efficacy. After administered with telitacicept 160 mg and 240 mg once weekly, the subjects showed a sustained decrease in QMG scores and MG clinical absolute scores from week 4 to week 24. On week 24, the subjects in telitacicept 160 mg group showed a reduction of 7.7 points on average in QMG score, and the subjects in telitacicept 240 mg group showed a reduction of 9.6 points on average in QMG score, compared with baseline. On week 24, the subjects in telitacicept 160 mg group showed a reduction of 13.8 points on average in MG clinical absolute score, and the subjects in telitacicept 240 mg group showed a reduction of 14.1 points on average in MG clinical absolute score, compared with baseline. On week 24, 92.9% of the subjects in telitacicept 160 mg group showed an improvement of ≥3 points in QMG scores, and 100% of the subjects in telitacicept 240 mg group showed an improvement of ≥3 points in QMG scores. In addition, both telitacicept 160 mg and telitacicept 240 mg significantly reduced the IgG, IgA, and IgM levels of the subjects.

### 6.6 Safety analysis

In this study, the administration of telitacicept 160 mg and telitacicept 240 mg once weekly had good safety in the treatment of patients with generalized myasthenia gravis. The severity of adverse events/adverse reactions was mostly mild and moderate (CTCAE grades 1 and 2). Only one subject in the telitacicept 160 mg group having adverse event of CTCAE grade 3 (decreased lymphocyte count). There were no adverse events/adverse reactions of CTCAE grades 4 and 5, no adverse events/adverse reactions leading to drug discontinuation, withdrawal and deaths, and no serious adverse reactions. During the study, there was one case of serious adverse event (infectious pneumonitis) in the telitacicept 160 mg group, which was considered by the investigator to be possibly unrelated to the tested drug. Therefore, the tested drug was not discontinued, and this case recovered after the treatment. The adverse events in this study were all expectable risks of telitacicept, and no new safety risks were identified.

### 6.7 Conclusion

In this study, telitacicept 160 mg and 240 mg showed good clinical efficacy and safety in the treatment of patients with generalized myasthenia gravis.

The above descriptions are only preferred embodiments, which are only examples and do not limit the combination of features necessary to implement the present invention. The headings provided are not intended to limit the various embodiments of the present invention. Terms such as "comprise," "contain," and "include" are not intended to be limiting. In addition, unless otherwise stated, the absence of a numerical modifier includes the plural, and "or" means "and/or". Unless otherwise defined herein, all technical and scientific terms used herein have the same meaning as commonly understood by those of ordinary skill in the art.

All publications and patents mentioned in the present application are incorporated herein by reference. Various modifications and variations of the methods and compositions described in the present invention without departing from the scope and spirit of the present invention will be apparent to those skilled in the art. Although the present invention has been described with specific preferred embodiments, it should be understood that the present invention as claimed should not be unduly limited to these specific embodiments. In fact, various modifications of the described modes for implementing the present invention which are apparent to those skilled in the relevant fields are intended to be included within the scope of the following claims.

## Claims

1. A method for treating myasthenia gravis, comprising administering a therapeutically effective amount of a TACI-Fc fusion protein to a subject in need thereof, wherein the TACI-Fc fusion protein comprises:
(i) a TACI extracellular region or a fragment thereof binding to Blys and/or APRIL; and
(ii) a fragment of a constant region of a human immunoglobulin.

2. The method according to claim 1, wherein the TACI extracellular region or the fragment thereof binding to Blys and/or APRIL comprises an amino acid sequence set forth in SEQ ID NO: 1.

3. The method according to claim 1, wherein the constant region of the human immunoglobulin comprises an amino acid sequence set forth in SEQ ID NO: 2 or an amino acid sequence having at least 90%, at least 91%, at least 92%, at least 93%, at least 95%, at least 96%, at least 97%, at least 98%, or at least 99% identity to SEQ ID NO: 2.

4. The method according to claim 3, wherein the constant region of the human immunoglobulin comprises one or more amino acids with a modification, and wherein the amino acid is selected from 3, 8, 14, 15, 17, 110, 111 or 173 position of SEQ ID NO: 2.

5. The method according to claim 4, wherein the modification is substitution, deletion or insertion of amino acid.

6. The method according to claim 5, wherein the substitution is selected from the group consisting of P3T, L8P, L14A, L15E, G17A, A110S, P111S and A173T.

7. The method according to claim 6, wherein the human immunoglobulin is IgG1, or the constant region of the human immunoglobulin comprises an amino acid sequence set forth in SEQ ID NO: 3.

8. The method according to claim 1, wherein the TACI-Fc fusion protein comprises an amino acid sequence set forth in SEQ ID NO: 4.

9. The method according to claim 9, wherein the TACI-Fc fusion protein is Telitacicept.

10. The method according to claim 9, wherein the TACI-Fc fusion protein is administered at a dose of 0.1 to 10 mg/kg each time.

11. The method according to claim 9, wherein the TACI-Fc fusion protein is administered at a dose of 160 to 240 mg each time, more preferably 160 mg or 240 mg each time.

12. The method according to claim 10 or 11, wherein the TACI-Fc fusion protein is administered 2-4 times per month and/or administered continuously for about 2-50 weeks.

13. The method according to claim 12, wherein the TACI-Fc fusion protein is administered subcutaneously, intramuscularly or intravenously, into a site on a thigh, an abdomen or an upper arm.

14. The method according to claim 13, wherein the myasthenia gravis is generalized myasthenia gravis (gMG), acetylcholine receptor antibody (AchR-Ab)-positive myasthenia gravis, muscle-specific receptor tyrosine kinase antibody (MuSK-Ab)-positive myasthenia gravis, or myasthenia gravis of class II or III according to the Myasthenia Gravis Foundation of America clinical classification.

15. The method according to claim 14, wherein the myasthenia gravis has a quantitative myasthenia gravis score (QMG) of≥ 8, and each of at least 4 items has a score of at least 2.

16. The method according to claim 15, wherein the subject is an adult or a child or has previously and stably received a standard treatment regimen for myasthenia gravis.

17. The method according to claim 16, wherein the standard treatment regimen includes: a cholinesterase inhibitor alone, a cholinesterase inhibitor in combination with a glucocorticoid or an additional immunosuppressant specified by the regimen, a glucocorticoid in combination with an additional immunosuppressant specified by the regimen, and a cholinesterase inhibitor in combination with a glucocorticoid and an additional immunosuppressant specified by the regimen.

18. A method for treating a subject with myasthenia gravis who has received a standard treatment regimen for myasthenia gravis, comprising (1) determining whether a subject has received a standard treatment regimen for myasthenia gravis, and (2) administering to the subject an effective amount of a TACI-Fc fusion protein if the subject has received a standard treatment regimen for myasthenia gravis.

19. The method according to claim 18, wherein the standard treatment regimen includes: a cholinesterase inhibitor alone, a cholinesterase inhibitor in combination with a glucocorticoid or an additional immunosuppressant specified by the regimen, a glucocorticoid in combination with an additional immunosuppressant specified by the regimen, and a cholinesterase inhibitor in combination with a glucocorticoid and an additional immunosuppressant specified by the regimen.

20. Use of a TACI-Fc fusion protein in the manufacture of a medicament for the treatment of a subject with myasthenia gravis.
